# EUROPEAN PATENT APPLICATION

(11) **EP 4 610 696 A1**
(43) Date of publication of application: **03.09.2025**
(21) Application number: 24159879.6
(22) Date of filing: 27.02.2024
(51) Int. Cl.: G01T 1/02, G01T 7/00

(54) **ELECTRONIC DEVICE AND METHOD FOR MONITORING AN EXPOSURE OF AN OPERATOR OF THE ELECTRONIC DEVICE**

(71) Applicant: Koninklijke Philips N.V., 5656 AG Eindhoven (NL)
(72) Inventor: VOGTMEIER, Gereon, 5656AG Eindhoven (NL); WEISS, Steffen, 5656AG Eindhoven (NL); LEUSSLER, Christoph Günther, 5656AG Eindhoven (NL); LIPS, Oliver, 5656AG Eindhoven (NL)
(74) Representative: Philips Intellectual Property & Standards

(57) **Abstract**

The invention concerns an electronic device, in particular a mobile phone, for monitoring an exposure of an operator of the electronic device, the electronic device comprising a radiation monitoring unit configured to measure ionizing radiation emitted in the environment of the electronic device, wherein the radiation monitoring unit is further configured to determine a dose result for the operator of the electronic device based on the measured ionizing radiation, the device further comprises an X-ray control unit configured for being in wireless communication with an X-ray system, wherein the X-ray control unit is configured to control the X-ray system based on the determined dose result for the operator of the device. Further, the invention concerns a method for monitoring an exposure of an operator of the device and a system for controlling an X-ray system comprising the electronic device.

## Description

### FIELD OF THE INVENTION

The present invention relates to the field of monitoring ionizing radiation. In particular, the present invention relates to the field of monitoring an exposure of an operator of an electronic device configured to control an X-ray system and a method of monitoring the exposure.

### BACKGROUND OF THE INVENTION

X-ray imaging operations are performed in proper shielded imaging rooms with interlock functionality at each door for limiting access to allowed persons only. The position of the patient, the position of the staff and the position of the operator is a well known parameter for these X-ray operations. Contrary thereto, for the operation of a mobile X-ray system the radiation protection and exposure measurement often are more complex compared to the defined and proper shielded imaging rooms. An option for measuring an exposure can be provided by mobile phones, which comprise a digital camera for capturing images or films. These mobile phones can be used for measurement of ionizing radiation. However, these single measurement of the ionizing radiation does not protect the operator of an X-ray system whether the measured exposure is too high and may not prevent the operator from being harmed when using the X-ray system.

### SUMMARY OF THE INVENTION

There may therefore be a need for an improved device and/or system for monitoring an exposure of an operator, wherein the device and/or system also allows a controlling of the X-ray system, at least partially, for improving the safety of the operator during using/operating the X-ray system.

An object of the invention is to provide an improved device and/or system that improves the security during critical situations of an operator using an X-ray system with respect to the ionizing radiation to which the operator is exposed during operation.

The object of the present invention is solved by the subject matter of the independent claims where further embodiments are incorporated in the dependent claims.

According to a first aspect of the invention, an electronic device, in particular a mobile phone, for monitoring an exposure of an operator of the electronic device is described. The electronic device comprising a radiation monitoring unit configured to measure ionizing radiation emitted in the environment of the electronic device, wherein the radiation monitoring unit is further configured to determine a dose result for the operator of the electronic device based on the measured ionizing radiation, an X-ray control unit configured for being in wireless communication with an X-ray system, wherein the X-ray control unit is configured to control the X-ray system based on the determined dose result for the operator of the device.

In other words, the present aspect of the invention relates to a device or apparatus configured for measuring electromagnetic radiation, in particular ionizing radiation such that the device may be configured to be used as a so called dosimeter and additionally may control an X-ray system at least partially and/or parts of the X-ray system at least partially. When controlling the X-ray system or parts thereof at least partially, in the context of the present invention is meant to at least control the access to the X-ray system, or to a part of it, like the X-ray tube. Beyond that, i.e. an extended control could be to control each single parameter of the X-ray system. This means, not only the access to the X-ray system but also the setting of the X-ray system. The electronic device allows to measure an exposure of the operator and based on this measurement is able to control the X-ray system. The measured radiation may be associated with the operator. This means the operator is considered to be exposed to the radiation measured by the electronic device, such that from the measured radiation the exposure, i.e. the dose result, may be determined. In other words, the device may be a personal dose detector and X-ray control unit to extend the safety functionality for staff and patients in a mobile setting where no extra shielded room is available. The device may also be applicable for patients comprising implanted seeds, such as radioactive material, wherein these patients have for example a brachytherapy and person which may be in contact with these patients.

In the present invention, the term "electronic device" shall be understood to describe any device which may be handheld and used by the operator and which device may, besides the hardware components such as the imager and/or camera, comprise software or software program products installed thereon for being able to perform the described monitoring of an exposure and for performing the method steps as described in another aspect of the present invention. For example, the device may be any apparatus which could be worn by the operator and/or held in the hand by the operator and/or held in a special holder for the electronic device, such as a mobile phone, laptop, or tablet. Further, when describing that the dose result is determined, this may encompass a processor and/or a processing unit which is used, hence configured, for determining the dose result. Alternatively, the processor may be used for the radiation monitoring unit, such that the monitoring unit may be configured to measure the radiation and does not determine/calculate the dose result. On the other hand, the processor and the radiation monitoring unit may interact with each other, and both may be used for determining, hence calculating, the dose result.

In the present invention, the term "radiation monitoring unit" shall be understood to describe a part of the device, which is able to measure the ionizing radiation emitted in the environment of the device. As the device is used by the operator, for example is handheld by the operator or worn in a pocket by the operator, the exposure of the device itself is equal to the exposure of the operator itself. The radiation monitoring unit may comprise at least one or more than one sensor for measuring the ionizing radiation for simple observation of the radiation the operator is exposed to. For example, the radiation monitoring unit may function as a so-called radiation dosimeter. Hence the radiation monitoring unit may be configured to measure uptake of external ionizing radiation, such that the device may be a personal dosimeter. The determined dose result according to the invention may simply be the measured ionizing radiation of the operator. The determined dose result may also be the measured dose since starting the device for measuring the ionizing radiation.

In the present invention, the term "in wireless communication" shall be understood to describe any communication done remotely, for example any wireless connection able to transmit data, such that both the electronic device and the X-ray system may be able to receive and transmit data, for exchange information, instructions and/or conditions. Such wireless communication may be any communication via a remote network, Bluetooth, a WLAN network, for example also via a hospital data network.

In the present invention, the term "X-ray system" shall be understood to describe any X-ray system used in a hospital or medical unit used for examination of patients. For example, a mobile X-ray system may be used. The device is not limited for being in contact with any specific X-ray system. The device may be in contact (at least the control unit) with different X-ray system, but not in parallel operation, but it may be able to communicate with different X-ray systems throughout a day. In other words, the device may be in wireless communication with various X-ray system which are used by the operator.

According to an exemplary embodiment of the invention, the device may further comprise a user management unit configured to identify whether the operator is a certified person allowed to access the X-ray system, and if the user management unit identifies the operator as the certified person, then the access to the X-ray control unit is provided to the operator for controlling the X-ray system. In other words, the X-ray control unit may be configured to perform, for example using a processor, a user management before allowing an access to an X-ray system. The user management may be based on a hospital (or any other applicable medical facility having X-ray device/systems) internal user management system comprising the registered and allowed user of any X-ray system, X-ray equipment of the hospital. The user management unit of the electronic device may have access to the user management of the hospital for identification of respective allowed user of the X-ray system(s). On the other hand, the user management unit may comprise the respective identification data on the electronic device itself.

A certified person may be any person which is allowed to operate an X-ray system, such as for example but not limited to a physician, medical assistants, medical specialist, and medical staff. Hence, the certified person is a person allowed to execute a scan with the X-ray system.

According to an exemplary embodiment of the invention, the identification of the operator as the certified person may comprise identification of the operator by at least one of password, fingerprint, and Face-ID, wherein the identification is not limited to these examples, other identification procedures applicable for identifying a person may be used.

According to an exemplary embodiment of the invention, the radiation monitoring unit may measure the ionizing radiation in real time and may be configured to determine the dose result for the operator in real time. This means that the exposure of the operator is monitored live during the performance of the X-ray system, such that for example, a live warning regarding a too high dose can be provided to the operator, and/or a live shut down of the X-ray system can be carried out. During the measurement of the ionizing radiation by the radiation monitoring unit the dose result may continuously be determined, such that any peak of the ionizing radiation may be realized, and the operator may be warned, and the X-ray system may be controlled based on the determined dose result. This allows, that any unwanted dose result may be recognized, and security measures can be taken directly.

According to an exemplary embodiment of the invention, the X-ray control unit may be configured to remotely control the X-ray system via the wireless communication. This means that the X-ray control unit may transmit and receive data and/or information which can be transmitted to the X-ray system. The X-ray control unit may be configured to remotely control any X-ray control unit to which the operator may have access as a certified person. The electronic device, hence, the X-ray control unit, may not necessarily be directly connected to the X-ray system as due to the wireless communication the access to the X-ray system may be provided even when the electronic device is in a distance to the X-ray system. The controlling of the X-ray system may, among other things, extend to access to the X-ray system, control parts of the X-ray system, for example the tube settings, and/or to control the whole X-ray system and its equipment. Due to the remote control by the X-ray control unit, the shut down of the X-ray system may be performed without interaction with the operator. Accordingly, if a too high dose result is determined the operator is immediately protected, as the X-ray control unit is in communication with the X-ray system and can cause the X-ray system to stop.

According to an exemplary embodiment of the invention, the radiation monitoring unit may be configured to indicate to the operator the determined dose result and if the dose result exceeds a predetermined threshold to warn the operator, and/or if the dose result exceeds a threshold the radiation monitoring unit causes the X-ray control unit to stop the X-ray system. Both the indication of the dose result and the warning of the operator may be performed independently from each other and are not necessarily connected to each other. The warning may be in any applicable form, which can be recognized by the operator. For example, any visible warning such as a blinking light, an acoustic warning such as an alarm tone, or a vibrational signal. These warning signs may be provided by the mobile phone to the operator. The predetermined threshold may be any preset threshold and/or limit for limiting the exposure of the operator to the ionizing radiation. For example, medical standards may apply for the threshold of a radiation dose, wherein these standards may be different depending on the respective country standards, and standards of the medical facilities (hospitals).

According to an exemplary embodiment of the invention, if the dose result may exceed the predetermined threshold prior to a beginning of the controlling of the X-ray system, the operator may not allowed to start the X-ray system, and/or if the dose result may exceed the predetermined threshold after controlling the X-ray system and after the start of the X-ray imaging, the X-ray control unit may interrupt the X-ray system, and/or if the dose result may be below the predetermined threshold the controlling of the X-ray system may be allowed and the X-ray system can start and/or proceed. These above-mentioned conditions may apply for one and the same threshold or each may apply for a respective different threshold, or any combination thereof. In other words, one threshold may trigger all the situations described above, wherein the resulting action may depend on the operating state of the X-ray system. As the electronic device may measure the ionizing radiation continuously the determined dose result may exceed the threshold before the operator may start a new X-ray imaging process. Further, if a failure with respect to a high radiation leak or the like in the X-ray system may occur prior to the beginning of an X-ray imaging process, the determined dose result may exceed the threshold and the electronic device limits the access to the X-ray system and the operator is prevented from starting the system and may be warned. Hence, this may result in an improvement of the safety of the X-ray imaging process. The same applies to the situation, if the X-ray imaging process has already been started but not finished and a too high radiation dose would occur, the electronic device may be able to warn the operator and to start a shut down of the X-ray system, such that the exposure of the operator is limited. In this case the X-ray imaging process is interrupted and not correctly finished, but the operator has been protected of too high ionizing radiation. On the other hand, if no risky dose is determined the access to the X-ray system is allowed and remains stable. The X-ray system must not undergo any interruption and the X-ray imaging process may continue correctly.

According to an exemplary embodiment of the invention, the device may further comprise a dose estimation unit configured to determine an estimated dose result after setting X-ray parameters via the X-ray control system, wherein the estimated dose result is determined based on the set X-ray parameters, if the estimated dose result after setting the X-ray parameters via the X-ray control system may exceed the predetermined threshold, the operator may not be allowed to start the X-ray system by the X-ray control unit. In particular, the estimation of the dose result may be a complex calculation involving the respective X-ray parameters, such as position of the tube to the patient, position of the tube to the operator, position of the operator to the patient. The X-ray parameters may be at least one of the following parameters, which need to be set prior to any X-ray imaging, such as tube current, a tube voltage, a distance of the patient to the tube, a distance of the operator to the tube, a setting of the X-ray collimator, a maximum dose setting of the X-ray tube. In other words, the dose estimation unit may receive the parameter configuration of the X-ray system, the operator wants to use. Further, the dose estimation unit calculates from the received parameter settings the estimated dose occurring during the X-ray imaging process and may determine together with the other features of the electronic device, whether this dose may be too high or whether it is below the predetermined threshold.

According to an exemplary embodiment of the invention, the electronic device may further comprise an interface configured to display to the operator at least one of the monitored ionizing radiation, the determined dose result, a type of connection to the X-ray system, a listing of necessary preparations for X-ray imaging to be carried out by the X-ray system, a cumulative dose of the determined dose result of the operator, and control parameters of the X-ray system. The interface may be used for the operator for interacting with the electronic device. For example, for indicating the dose result, on the other hand the interface may be used by the operator for remotely interacting, hence controlling, the X-ray system in wireless communication. In other words, the interface may be a control panel for remotely controlling the X-ray system to which the electronic device is connected.

According to an exemplary embodiment of the invention, if the determined dose result exceeds the predetermined threshold, the X-ray control unit may be configured to adjust at least one X-ray imaging condition before starting the X-ray system. In the context of the present invention an X-ray condition may be understood to describe at least one of a position of the operator with respect to the X-ray source, a number of X-ray imaging scans; a distance of the operator with respect to the X-ray source; at least one X-ray parameter such as voltage, current, a time of radiation, and an identification of the X-ray operator. The X-ray control unit may also adjust more than one X-ray imaging condition depending on which condition and how many conditions have to be corrected. For instance, if the radiation monitoring unit determines that the dose result would exceed the predetermined threshold, the X-ray control unit may verify that with the amendment of the X-ray imaging condition the dose result would not exceed the threshold, such that a change of at least one conditions may help to allow the operator to start the X-ray imaging process. For instance, for the estimated dose result, the X-ray control unit may verify that an X-ray parameter may be set or reset, such as the tube current or voltage and after the reset of this parameter the dose result would be in an allowable range for the operator. Further, during the use of the electronic device the dose result may exceed the threshold because the distance between operator and the tube is too low, the X-ray control unit may indicate and/or may adjust the distance between the operator and the X-ray tube by rearranging the tube. This rearrangement of the tube may be performed by manually positioning of the tube or the operator may move the tube.

According to an exemplary embodiment of the invention, the ionizing radiation may be X-ray radiation emitted from the X-ray system used by the operator, wherein the radiation monitoring unit may determine the dose result from the received X-ray radiation. The electronic device and the radiation monitoring unit may be adapted to the specific X-ray radiation as this may be the most relevant radiation for the operator. Hence, the electronic device may be calibrated for the X-ray radiation for achieving proper exposure monitoring results.

According to an exemplary embodiment of the invention, the device may be in communication with a database on which a personal dose of the operator is stored and/or wherein the device may comprises a storage for storing the personal dose of the operator, wherein the determined dose result is stored in the storage and/or is communicated to the database, wherein the dose result and the personal dose of the operator may be compared by the radiation monitoring unit.
In other words, the radiation dose monitoring unit may store the dose result of the operator in an internal and/or external database or any combination thereof. The database may store the actual measured dose of the electronic device and may store personal dose of the operator, such that a comparison with the threshold may be carried out.

According to an exemplary embodiment of the invention, the radiation monitoring unit may comprise an ionizing radiation sensitive detector. The radiation monitoring unit may also comprise more than one radiation sensitive detector. The detector and the radiation monitoring unit may be used by a respective software application of the electronic device. In other words, the detector together with the radiation monitoring unit, which determines the dose result, may be used as a person dosimeter.

According to an exemplary embodiment of the invention, the ionizing radiation sensitive detector may be a camera of the mobile phone comprising an image sensor sensitive of the ionizing radiation. The camera of the mobile phone may be the radiation sensitive detector and a corresponding application of the mobile phone may be used and may be provided on the electronic device for processing the signals received through the camera. In particular, the image sensor of the camera may be a CMOS (Complementary Metal Oxide Semiconductor) image sensor is sensitive to ionizing radiation.

According to an exemplary embodiment of the invention, the device may be configured to determine a cumulative dose result of the operator over time based on the radiation measured by the radiation measurement unit. The cumulative dose result is the overall dose result resulting over a certain period of time. These cumulative dose result should also not exceed a predetermined threshold. The cumulative dose result may be used to indicate to the operator whether he will be allowed to perform further X-ray imaging. As the electronic device may be used as a personal dosimeter the cumulative dose result would be summed up for the operator using e.g. at one day several different X-ray devices/systems but it could also be a combination when the person is part of the time operator but in other cases an interventional room and also get some dose - but the operator would be mot in control of the system (however the operator may get a waring then).

According to a second aspect of the invention, a system for controlling an X-ray system is described. The system comprising an electronic device configured for monitoring ionizing radiation according to any of the embodiments referring to the electronic device. The system further comprises an X-ray system configured for receiving data from the electronic device, wherein the X-ray system is controllable by the electronic device. The electronic device included in the system may comprise any of the embodiments described with the electronic device hereinabove. For example, the system may be a mobile phone, as the electronic device, and a X-ray system, wherein the mobile phone may be used to measure the exposure of the operator and based on this information is able to control at least one of the access, X-ray parameters, start and stop of the X-ray system. The mobile phone and the X-ray system are in wireless communication, such that the mobile phone may control the X-ray system remotely.

According to a third aspect of the invention, a method of monitoring an exposure of an operator is described. The method comprising the steps of providing an electronic device for monitoring the exposure, measuring ionizing radiation emitted in the environment of the device using a radiation monitoring unit, determining a dose result for the operator of the device based on the measured ionizing radiation by the radiation monitoring unit, being in wireless communication with an X-ray system using an X-ray control unit of the device, and controlling the X-ray system via the wireless communication based on the determined dose result for the operator using the X-ray control unit. Furthermore, the embodiments described with the electronic device may be applied to the method. Therefore, the method may among other things, further comprise the steps of: identifying whether the operator is a certified person allowed to access the X-ray system by a user management unit, and if the user management unit identifies the operator as the certified person, then providing access to the X-ray control unit for the operator for controlling the X-ray system; measuring the ionizing radiation in real time; determining the dose result for the operator in real time; warning the operator if the dose result exceeds a predetermined threshold and stopping the X-ray system if the dose result exceeds a threshold; adjusting at least one X-ray imaging condition before starting the X-ray system, if the determined dose result exceeds the predetermined threshold; communicate with a database on which a personal dose of the operator is stored and/or wherein the device comprises a storage for storing the personal dose of the operator, wherein the determined dose result is stored in the storage and/or is communicated to the database, wherein the dose result and the personal dose of the operator may be compared by the radiation monitoring unit.

According to a fourth aspect of the invention a use of a mobile phone for monitoring an exposure of an operator is described, wherein the mobile phone is used for measuring ionizing radiation emitted in the environment of the electronic device by a radiation monitoring unit, wherein the radiation monitoring unit is further configured to determine a dose result for the operator of the electronic device based on the measured ionizing radiation. The mobile phone is further used for being in wireless communication with an X-ray system by an X-ray control unit, wherein the X-ray control unit is configured to control the X-ray system based on the determined dose result for the operator of the device.

It has to be noted that embodiments of the invention are described with reference to different subject matters. In particular, some embodiments are described with reference to method type claims whereas other embodiments are described with reference to the apparatus type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

### BRIEF DESCRIPTION OF THE DRAWINGS

The aspects defined above, and further aspects of the present invention are apparent from the examples of embodiment to be described hereinafter and are explained with reference to the examples of embodiment. The invention will be described in more detail hereinafter with reference to examples of embodiment but to which the invention is not limited.
Fig 1 illustrates schematically an electronic device, in particular a mobile phone, for monitoring an exposure of operator of the electronic device according to an exemplary embodiment of the invention.
Fig. 2 illustrates schematically features of an electronic device according to an exemplary embodiment of the invention.
Fig. 3 illustrates a flow diagram showing steps for a method for monitoring an exposure of an operator according to an exemplary embodiment of the invention.

### DETAILED DESCRIPTION OF EMBODIMENTS

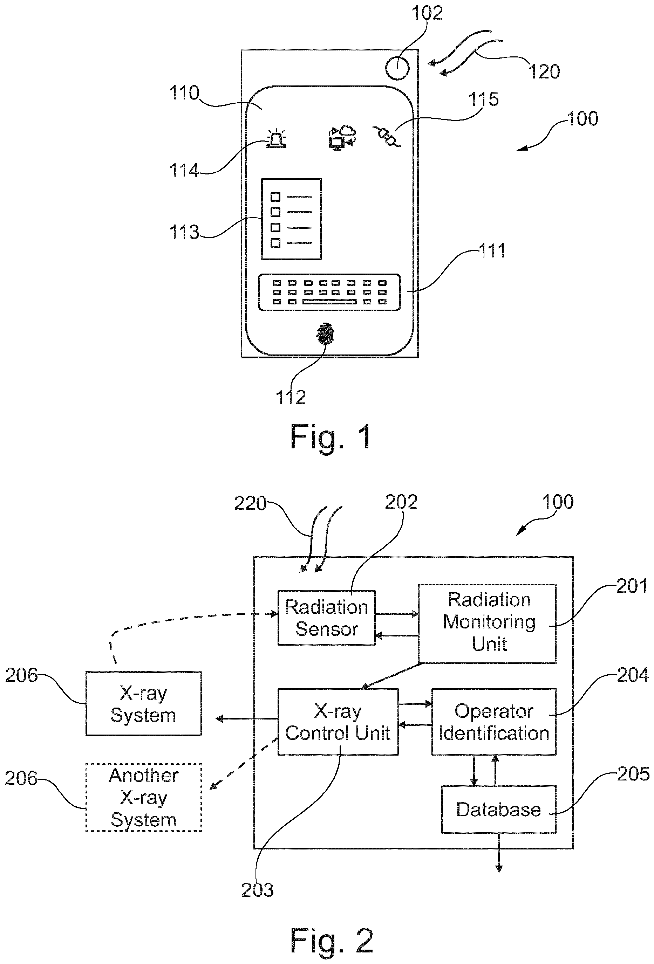

Fig 1 illustrates schematically an electronic device 100, in particular a mobile phone, for monitoring an exposure of operator of the electronic device 100 according to an exemplary embodiment of the invention. The electronic device 100 illustrated in Fig. 1 is a mobile phone, wherein the mobile phone 100 comprises the radiation monitoring unit, here illustrated as the camera 102 of the mobile phone 100. This radiation monitoring unit, the camera 102, is able to measure the occurring radiation 120 in the environment of the electronic device 100 and is configured to determine a dose result for the operator of the electronic device 100 based on the measured ionizing radiation 120. The X-ray control unit (not visible in Fig. 1) is a part of the electronic device 100 and is in wireless communication with an (external) X-ray system (not illustrated in Fig. 1). The X-ray control unit is configured to control the X-ray system based on the determined dose result for the operator of the device 100. The device 100 may be a handheld, portable device 100, such that the operator is able to carry the device 100 easily in his pocket or elsewhere. The identification of the operator and whether the operator is a certified person allowed to access the (external) X-ray system can be carried out by a user management system. This user management system may verify via an identifier 112, such as a fingerprint of the operator, whether the operator is a certified person. If the user management unit identifies the operator via the identifier 112 as the certified person, then the access to the X-ray control unit may be provided to the operator for controlling the X-ray system. The radiation monitoring unit is configured to indicate to the operator the determined dose result 113 and if the dose result 113 exceeds a predetermined threshold to warn the operator, and/or if the dose result 113 exceeds a threshold the radiation monitoring unit causes the X-ray control unit to stop the X-ray system. In Fig. 1 the dose result 113 may be presented on an interface 110, such as a display, to the operator in form of a list. This list may show the dose results 113 from last measurements over the day or over any other period of time. The indication of this information is not limited to this special list, all other visible indicators may be used to provide the information about the dose result 113 to the operator. The warning of the operator may be provided visually via a visual indicator 114, such as a light or pictogram on the interface 110 of the mobile phone 100. The warning may also be provided acoustically to the operator. The interface 110 may also provide further information to the operator, for example with visual indicators 115 showing whether access to the X-ray system is granted or whether access to a database may be provided. The interface 110 may also be used to remotely adjust parameters of the X-ray system via an input 111.

Fig 2 illustrates schematically an electronic device 100 according to an exemplary embodiment of the invention. The device 100 comprises the radiation monitoring unit 201, the X-ray control unit 203 and the user management unit 204 used as the operator identification 204. Further the device 100 comprises an internal database 205, wherein this database 205 may be in contact with an external database or other apparatuses or software system for exchanging data. The radiation monitoring unit 201 monitors, hence measures, via the radiation sensor 202 the ionizing radiation 220 in the environment of the device 100, hence in the environment of the operator. The detected ionizing radiation 220 is provided to the radiation monitoring unit 201, which determines a dose result from the ionizing radiation 220. The determined dose result is provided to the X-ray control unit 203 which is able to control the X-ray system 206 based on the determined dose result. The X-ray control unit 203 is able to be in wireless communication with more than one X-ray system. For example, if the operator uses different X-ray systems the X-ray control unit 203 is able to provide access to all of these different X-ray systems 206 and is further configured to identify whether the operator is a certified person for each of these different X-ray systems 206. The identification, whether the operator is a certified person is carried out by the user management unit 204. The arrow in Fig. 2 from the X-ray system 206 to the radiation sensor 202 should indicate the influence of the X-ray system(s) on the measured radiation 220.

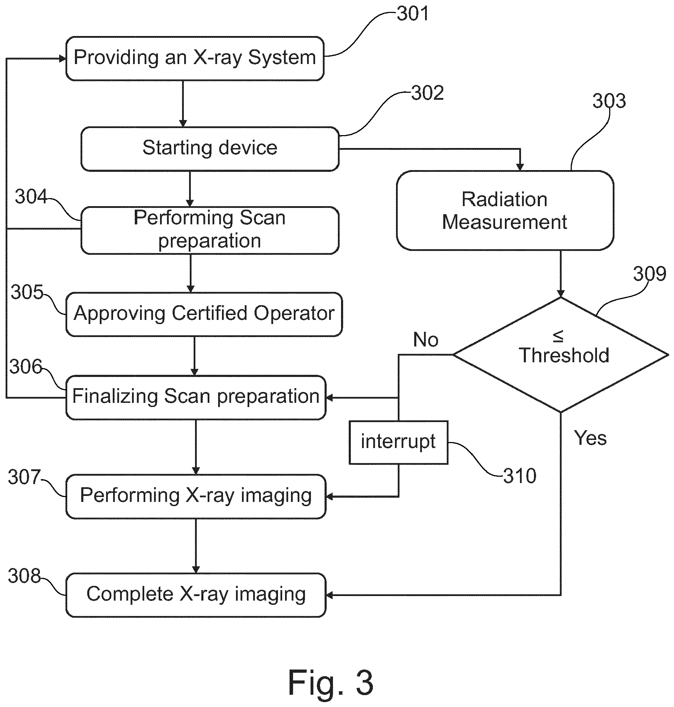

Fig 3 illustrates a flow diagram showing steps for a method for monitoring an exposure of an operator according to an exemplary embodiment of the invention. The flow diagram in Fig. 3 illustrates the different constraints when exceeding a threshold of the dose result. This flow diagram illustrates the process the electronic device 100 is configured to perform and also illustrates the method for monitoring an exposure of the operator. The steps in Fig. 3 will be described with reference to the method, but these steps may also apply to the process carried out by the features of the electronic device 100 as described in the different embodiments hereinabove. In the first step 301 an X-ray system is provided, for example a mobile X-ray system is provided to a patient. Additionally, or simultaneously the electronic device 100 is provided to the operator. Advantageously the operator is carrying the electronic device with him. In the next step 302 the electronic device is started, maybe the electronic device is running all the time, so this step may be obsolete. By the starting of the electronic device the radiation measurement in step 303 is started automatically. The radiation measurement may also be started separately by the operator. In the next step 304 the preparations for an X-ray image scan are performed. This step may include the setting of the X-ray parameters, wherein the setting may be performed by the operator on the X-ray system or via the electronic device 100 and is transmitted via wireless communication to the X-ray system. In the next step 305 the access to the X-ray system will be checked, hence the operator is identified as certified person for having access to the X-ray system. Step 304 and 305 may be changed in their order, such that step 305 may be performed before step 304. During the steps 302 to steps 308, the completing of the X-ray imaging process, the radiation monitoring unit determines whether the determined dose result exceeds a predetermined threshold, see 309. This verification is carried out during the period of time the electronic device 100 has been started, such that during every step 301 to 308 the electronic device 100 is able to monitor the ionizing radiation. If the determined dose result would exceed the threshold 309 the X-ray control unit would interrupt 310 the X-ray system and all possible running scans and operations of the X-ray system. The X-ray system may be stopped by the X-ray control unit of the electronic device, if the dose result exceeds the predetermined threshold 309 prior to a beginning of the controlling of the X-ray system, the operator is not allowed to start the X-ray system. For example, before the scan preparation, step 306, has been finalized the access to the X-ray system is denied or interrupted. If the dose result exceeds the predetermined threshold 309 after controlling the X-ray system and after the start of the X-ray imaging (X-ray imaging has been started in step 307), the X-ray control unit interrupts the X-ray system. Hence the X-ray control unit would interrupt during step 307. If the dose result is below the predetermined threshold the controlling of the X-ray system is allowed and the X-ray system can start and/or proceed. The device may further comprise a dose estimation unit (not illustrated) configured to determine an estimated dose result after setting X-ray parameters via the X-ray control system (which is done in step 304), wherein the estimated dose result is determined based on the set X-ray parameters, and if the estimated dose result after setting the X-ray parameters via the X-ray control system exceeds the predetermined threshold 309, the operator is not allowed to start the X-ray system by the X-ray control unit. Hence, the X-ray control unit interrupts the X-ray system before finalizing the scan preparation in step 306. The steps of the herein described process/method may be changed in their order, hence the described order is not limited to this specific order. If the dose result does not exceed the predetermined threshold 309 the scan preparation can be finalized, step 306, the X-ray imaging can be performed, step 307, and the X-ray imaging process can be completed.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfill the functions of several items re-cited in the claims. The mere fact that certain measures are re-cited in mutually different dependent claims does not indicate that a combination of these measures cannot be used to advantage. Any reference signs in the claims should not be construed as limiting the scope.

### LIST OF REFERENCE SIGNS:

- 100, 200: electronic device
- 110: interface
- 111: input
- 112: identifier
- 113: dose result
- 114: indicator
- 115: indicator
- 120, 220: ionizing radiation
- 201: radiation monitoring unit
- 202: radiation sensor
- 203: X-ray control unit
- 204: user management unit
- 205: database
- 206: X-ray system
- 301 to 310: process/method steps

## Claims

1. An electronic device, in particular a mobile phone, for monitoring an exposure of an operator of the electronic device, the electronic device comprising
a radiation monitoring unit configured to measure ionizing radiation emitted in the environment of the electronic device,
wherein the radiation monitoring unit is further configured to determine a dose result for the operator of the electronic device based on the measured ionizing radiation,
an X-ray control unit configured for being in wireless communication with an X-ray system,
wherein the X-ray control unit is configured to control the X-ray system based on the determined dose result for the operator of the device.

2. The electronic device according to claim 1, further comprising:
a user management unit configured to identify whether the operator is a certified person allowed to access the X-ray system, and
if the user management unit identifies the operator as the certified person, then the access to the X-ray control unit is provided to the operator for controlling the X-ray system.

3. The electronic device according to claim 1 or 2, wherein
the radiation monitoring unit measures the ionizing radiation in real time and is configured to determine the dose result for the operator in real time.

4. The electronic device according to any of the preceding claims,
wherein the X-ray control unit is configured to remotely control the X-ray system via the wireless communication.

5. The electronic device according to any of the preceding claims,
wherein the radiation monitoring unit is configured to indicate to the operator the determined dose result and if the dose result exceeds a predetermined threshold to warn the operator, and/or
if the dose result exceeds a threshold the radiation monitoring unit causes the X-ray control unit to stop the X-ray system.

6. The electronic device according to the preceding claim 5, wherein
if the dose result exceeds the predetermined threshold prior to a beginning of the controlling of the X-ray system, the operator is not allowed to start the X-ray system,
if the dose result exceeds the predetermined threshold after controlling the X-ray system and after the start of the X-ray imaging, the X-ray control unit interrupts the X-ray system,
if the dose result is below the predetermined threshold the controlling of the X-ray system is allowed and the X-ray system can start and/or proceed.

7. The electronic device according to any of the preceding claims 5 or 6, wherein the device further comprises
a dose estimation unit configured to determine an estimated dose result after setting X-ray parameters via the X-ray control system, wherein the estimated dose result is determined based on the set X-ray parameters,
if the estimated dose result after setting the X-ray parameters via the X-ray control system exceeds the predetermined threshold, the operator is not allowed to start the X-ray system by the X-ray control unit.

8. The electronic device according to any of the preceding claims 5 to 7, wherein
if the determined dose result exceeds the predetermined threshold, the X-ray control unit is configured to adjust at least one X-ray imaging condition before starting the X-ray system.

9. The electronic device according to any one of the preceding claims,
wherein the ionizing radiation is X-ray radiation emitted from the X-ray system used by the operator, wherein the radiation monitoring unit determines the dose result from the received X-ray radiation.

10. The electronic device according to any one of the preceding claims,
wherein the device is in communication with a database on which a personal dose of the operator is stored and/or wherein the device comprises a storage for storing the personal dose of the operator,
wherein the determined dose result is stored in the storage and/or is communicated to the database,
wherein the dose result and the personal dose of the operator may be compared by the radiation monitoring unit.

11. The electronic device according to any one of the preceding claims,
wherein the radiation monitoring unit comprises an ionizing radiation sensitive detector.

12. The electronic device according to any one of the preceding claims,
wherein the ionizing radiation sensitive detector is a camera of the mobile phone comprising an image sensor sensitive of the ionizing radiation.

13. The electronic device according to any one of the preceding claims,
wherein the device is configured to determine a cumulative dose result of the operator over time based on the radiation measured by the radiation measurement unit.

14. System for controlling an X-ray system, comprising:
an electronic device configured for monitoring ionizing radiation according to any of the claims 1 to 13,
an X-ray system configured for receiving data from the electronic device,
wherein the X-ray system is controllable by the electronic device.

15. Method of monitoring an exposure of an operator, the method comprising the steps of
providing an electronic device for monitoring the exposure,
measuring ionizing radiation emitted in the environment of the device using a radiation monitoring unit,
determining a dose result for the operator of the device based on the measured ionizing radiation by the radiation monitoring unit,
being in wireless communication with an X-ray system using an X-ray control unit of the device,
controlling the X-ray system via the wireless communication based on the determined dose result for the operator using the X-ray control unit.
